# EUROPEAN PATENT APPLICATION

(11) **EP 2 460 892 A2**
(43) Date of publication of application: **06.06.2012**
(21) Application number: 10803952.0
(22) Date of filing: 30.07.2010
(51) Int. Cl.: C12Q 1/68, G01N 33/53

(54) **METHOD AND KIT FOR PROGNOSIS MANTLE CELL LYMPHOMA**

(30) Priority: 31.07.2009 ES 200901738
(71) Applicant: Hospital Clínic de Barcelona, 08036 Barcelona (ES); Institut d'Investigacions Biomédiques August Pi i Sunyer, 08036 Barcelona (ES); Fundació Clínic per a la Recerca Biomèdica, 08036 Barcelona (ES); Universidad de Barcelona, 08028 Barcelona (ES)
(72) Inventor: CAMPO GÜERRI, Elías, E-08015 Barcelona (ES); SALAMERO GARCÍA, Olga, E-08011 Barcelona (ES); FERNÀNDEZ PASCUAL, Verònica, E-08940 Cornellà de Llobregat (ES); JARES GERBOLES, Pedro, E-08042 Barcelona (ES); LÓPEZ GUILLERMO, Armando, E-08017 Barcelona (ES)
(74) Representative: ZBM Patents
(86) International application number: PCT/ES2010/070531
(87) International publication number: WO 2011/012763

(57) **Abstract**

The method and the kit are useful as tools for classifying a patient diagnosed with mantle cell lymphoma into the category of: indolent or conventional. The method comprises: a) providing a sample from a patient suffering from mantle cell lymphoma; b) determining the level of expression of at least one gene selected from the group consisting of: RNGTT, HDGFRP3, FARP1, HMGB3, LGALS3BP, PON2, CDK2AP1, DBN1, CNR1, CNN3, SOX11, SETMAR and CSNK1E in said sample; and c) comparing the level of expression of each of the measured genes with respect to the level of expression of the same genes in a control sample; wherein the absence of expression or the underexpression of said genes with respect to the same genes in said control sample is indicative of the indolent clinical course of the MCL.

## Description

The present invention relates to methods for the prognosis of cancer, particularly for the prognosis of mantle cell lymphoma, and more particularly for the prognosis of an indolent type of mantle cell lymphoma.

### BACKGROUND ART

Conventional Mantle Cell Lymphoma (MCL) is an aggressive lymphoid neoplasm with a rapid clinical evolution, short response to therapy, frequent relapses and a median survival of 3-4 years. The aggressive biological behaviour of this lymphoma has been attributed to the peculiar genetic and molecular mechanisms involved in its pathogenesis that combine the constitutive deregulation of cell proliferation due to the t(11;14)(q13;q32) and cyclin D1 over-expression, with a high level of chromosomal instability related to the disruption of the DNA damage response pathway and activation of cell survival mechanisms. Patients suffering from MCL are treated with different strategies including intensive chemotherapy regimens but, unfortunately, few patients may be cured with current therapies.

In addition to the well recognised molecular hallmarks for MCL diagnosis, namely t(11;14) translocation and cyclin D1 over-expression, some authors have recently associated MCL with other molecular markers. Wang et al. (cf. "The subcellular Sox11 distribution pattern identifies subsets of mantle cell lymphoma: correlation to overall survival", British Journal of Haematology 2008, vol. 143, p. 248-52) report the over-expression of SOX11 in MCL cells, while Ortega-Paino *et al.* report the over-expression of HDGFRP3 in MCL (cf. "Functionally associated targets in mantle cell lymphoma as defined by DNA microarrays and RNA interference", Blood 2008, vol. 111, p. 1617-24).

Recent studies have identified a group of patients diagnosed with MCL that follow a very indolent clinical course with a long survival of more than 7-10 years, some of them not needing chemotherapeutic treatment (cf. Espinet B. et al., "Clonal proliferation of cyclin D1-positive mantle lymphocytes in an asymptomatic patient: an early-stage event in the development or an indolent form of a mantle cell lymphoma?", Human Pathology 2005, vol. 36, p. 1232-7; Swerdlow S.H. et al., "From centrocytic to mantle cell lymphoma: a clinicopathologic and molecular review of 3 decades", Human Pathology 2002, vol. 33, p. 7-20). Small subsets of patients with a similar long evolution have been also recognized in most clinical studies of this tumour (cf. Orchard J. et al., "A subset of t(11;14) lymphoma with mantle cell features displays mutated IgVH genes and includes patients with good prognosis, nonnodal disease", Blood 2003, vol. 101, p. 4975-81; Martin P, et al., "Outcome of deferred initial therapy in mantle-cell lymphoma", J. Clin. Oncol. 2009, vol. 27, p. 1209-13).

Nowadays, the available diagnostic tools allow for the identification of patients suffering from MCL but they can not provide information about whether the illness courses in an indolent way.

Therefore, there is the need of providing means for the identification of a patient suffering from indolent cancer cell lymphoma.

### SUMMARY OF THE INVENTION

The inventors have found that a set of genes are underexpressed or not expressed in tumor cells from patients suffering from a mantle cell lymphoma with an indolent clinical course.

There are recent documents in the state of the art that disclose a relationship between the expression of certain genes and MCL. Among them, Wang *et al* (*supra*) report a direct relationship of SOX11 over-expression and the diagnosis of MCL. In fact, many authors have regarded SOX11 as a highly reliable biomarker for the diagnosis of MCL. Similarly, other authors have reported the over-expression of HDGFRP3 in MCL (see Ortega-Paino *et al., supra*), and that its down-regulation would inhibit tumor proliferation.

Surprisingly, the inventors of the present invention have observed that SOX11 and HDGFRP3, among other genes, are not expressed in MCL cells extracted from patients diagnosed with MCL with an indolent course of the disease.

Thus, the first aspect of the present invention provides a method for the prognosis of a mantle cell lymphoma with an indolent clinical course, comprising the steps of: a) providing a sample from a patient suffering from mantle cell lymphoma; b) determining the level of expression of at least one gene selected from the group consisting of: RNGTT, HDGFRP3, FARP1, HMGB3, LGALS3BP, PON2, CDK2AP1, DBN1, CNR1, CNN3, SOX11, SETMAR and CSNK1E in said sample; and c) comparing the level of expression of each of the measured genes with respect to the level of expression of the same genes in a control sample; wherein the absence of expression or the underexpression of said genes with respect to the same genes in said control sample is indicative of the indolent clinical course of the mantle cell lymphoma.

As it is shown below, the set of genes of the method of the present invention have a remarkable differential expression pattern in a sample from a patient with MCL with an indolent clinical course when compared with a sample from a patient suffering from conventional MCL. Particularly, the inventors have found that said genes are overexpressed in cells derived from conventional MCL and underexpressed or not expressed in iMCL. This differential expression pattern makes the method according to the first aspect of the invention a valuable tool which allows distinguishing with high precision between the two forms of MCL, the conventional (cMCL, which is related to a rapid, aggressive disease progression) and the indolent (iMCL, which is related to less aggressive disease progression).

Until now, the available tools in the state of the art have been for the diagnosis of MCL as a highly aggressive lymphoma. In this way, when the subject was diagnosed of MCL he was submitted to chemotherapy (which is the most aggressive treatment in order to avoid the tumor progression).

Advantageously, the method of the present invention can be used to identify those patients with an iMCL, which are characterized by having a slow evolution, as compared to patients with cMCL, which are characterized by a highly aggressive tumor. In other words, the method of the invention can be used as a tool for classifying a patient diagnosed with MCL into the category of indolent or conventional. Patients suffering from an indolent form of MCL have a longer survival and do not need to be treated with an aggressive chemotherapy while patients with a cMCL need to be subjected immediately to an intensive therapy. Up to date, the indolent form of the disease could not be predicted in any way, thus resulting in the administration of aggressive therapies to patients that would not need them and causing them unnecessary suffering. Consequently, the method of the invention is of importance for the field of cancer therapy because it will enable the medical community to apply risk-adapted treatment strategies to patients suffering from MCL.

As the control sample of step (c), the inventors have used tumoral cells extracted from patients that have been diagnosed with cMCL, said patients needing for therapy because of rapid progression (following the standard criteria described by Cheson B. D. et al., "Revised Response Criteria for Malignant Lymphoma", J. Clin. Oncol., 2007, vol. 25, p. 579-586).

By "prognosis" it is included the act or process of predicting the probable course and outcome of a lymphoma, e.g. determining survival probability.

SOX11 (SRY (sex determining region Y)-box 11) encodes a member of the SOX family of transcription factors involved in the regulation of embryonic development and in the determination of the cell fate. Proteins are grouped into this family if they contain a DNA-binding HMG-domain (High Mobility Group) with 25 strong amino acid similarity (usually >50%) to the HMG domain of Sry, a sex determining protein. The gene sequence is available at the Genbank database with the reference number NM_003108. The protein encoded by SOX11 may act as a transcriptional regulator after forming a protein complex with other proteins. The protein may function in the developing nervous system and tumorigenesis. There are recent documents in the state of the art that disclose a direct relationship between the overexpression of SOX11 and the diagnosis of MCL. Surprisingly, as shown in the examples bellow, the inventors have observed that SOX11 is not expressed in MCL cells extracted from patients with an indolent course of the disease.

HDGFRP3 (hepatoma-derived growth factor, related protein 3, Genbank reference number NM_016073.2) is a member of the hepatoma-derived growth factor (HDGF) family. The HDGFs all share a common sequence motif in their N-terminal domain, called the HATH (homologous to the amino terminus of HDGF) region. The HDGF has been reported to stimulate growth of fibroblasts and some hepatoma cells. Previous authors have reported the overexpression of HDGFRP3 in MCL, and that its down-regulation inhibits proliferation. However, the inventors have surprisingly observed that cells extracted from a MCL which has an indolent clinical course, have no expression for this gene.

CNN3 (calponin 3, Genbank reference number NP_001830.1) encodes a protein with a markedly acidic C terminus that is associated with the cytoskeleton but is not involved in contraction.

FARP1 (FERM, RhoGEF (ARHGEF) and pleckstrin domain protein (chondrocyte-derived), Genbank reference number NM_001 001715) was originally isolated through subtractive hybridization due to its increased expression in differentiated chondrocytes versus dedifferentiated chondrocytes. The resulting protein contains a predicted ezrin-like domain, a Dbl homology domain, and a pleckstrin homology domain. It is believed to be a member of the band 4.1 superfamily whose members link the cytoskeleton to the cell membrane. Two alternatively spliced transcript variants encoding distinct isoforms have been found for this gene.

PON2 (paraoxonase 2, GenBank reference number NM_000305) encodes a member of the paraoxonase gene family, which includes three known members located adjacent to each other on the long arm of chromosome 7. The encoded protein is ubiquitously expressed in human tissues, membrane-bound, and may act as a cellular antioxidant, protecting cells from oxidative stress. Hydrolytic activity against acylhomoserine lactones, important bacterial quorum-sensing mediators, suggests the encoded protein may also play a role in defence responses to pathogenic bacteria. Mutations in this gene may be associated with vascular disease and a number of quantitative phenotypes related to diabetes.

DBN1 (drebrin 1, GenBank reference number NM_004395) encodes a cytoplasmic actin-binding protein thought to play a role in the process of neuronal growth. It is a member of the drebrin family of proteins that are developmentally regulated in the brain.

RNGTT (RNA guanylyltransferase and 5'-phosphatase, GenBank reference number NM_003800) encodes an enzyme reported to have mRNA capping and protein amino acid dephosphorylation activities.

CSNK1E (Casein kinase 1 epsilon, GenBank reference number NM_001894) encodes a serine/threonine protein kinase and a member of the casein kinase I protein family, whose members have been implicated in the control of cytoplasmic and nuclear processes, including DNA replication and repair.

SETMAR (SET domain and mariner transposase fusion gene, GenBank reference number NM_006515) has been reported to be involved in DNA repair and transposition.

HMGB3 (High-mobility group box 3, GenBank reference number NM_005342) belongs to the high mobility group (HMG) protein superfamily. Like HMG1 (MIM 163905) and HMG2 (MIM 163906), HMGB3 contains DNA-binding HMG box domains and is classified into the HMG box subfamily. Members of the HMG box subfamily are thought to play a fundamental role in DNA replication, nucleosome assembly and transcription.

LGALS3BP (Lectin, galactoside-binding, soluble, 3 binding protein, GenBank reference number NM_005567) appears to be implicated in immune response associated with natural killer (NK) and lymphokine-activated killer (LAK) cell cytotoxicity.

CDK2AP1 (CDK2-associated protein 1, GenBank reference number NM_004642) encodes a protein which is thought to negatively regulate CDK2 activity by sequestering monomeric CDK2, and targeting CDK2 for proteolysis. This protein was found to also interact with DNA polymerase alpha/primase and mediate the phosphorylation of the large p180 subunit, which suggested the regulatory role in DNA replication during S phase of the cell cycle.

CNR1 (Cannabinoid receptor type 1, GenBank reference number NM_016083) encodes a protein that is found in the brain and is activated by the psychoactive drug cannabis and by a group of endocannabinoid neurotransmitters. The protein is known to be involved in lipogenesis, gastrointestinal activity, cardiovascular activity and pain.

In one embodiment of the first aspect of the invention at least one of the genes is selected from the group consisting of: SOX11, FARP1, PON2, HDGFRP3, CNN3, DBN1, and HMGB3. As shown in the examples below, these genes are more differently expressed in iMCL cells as compared with cMCL cells. Among said genes, the best differentially expressed genes are SOX11, HDGFRP3 and CNN3. More preferably, the method of the present invention is carried out determining the level of expression of SOX11.

According to the present invention, the prognosis of iMCL can be performed by determining the expression of at least one of the above mentioned genes. In this way, the person skilled in the art may choose to determine: (a) the expression of only one of the above genes, (b) the expression of several genes, or (c) the expression of all the above mentioned genes. Preferably, the expression of the genes disclosed in the preferred embodiments will be determined. For instance, the person skilled in the art can choose to determine the expression of SOX11 and CNN3, on their own or together with one or more genes of the list of 13 genes mentioned above. The method of the invention could be also carried out by determining the expression of the genes SOX11, FARP1, PON2, CNN3, DBN1 and HMGB3. It will also be apparent to the person skilled in the art that the method of the present invention can be used together with other diagnosis or prognosis clinical, pathological or molecular markers to complement the information obtained by said biomarkers in the diagnosis or prognosis of MCL.

In the sense of the present invention, qualitative, semi-quantitative or quantitative determinations of the expression level of the disclosed genes can be performed. The level of expression of a gene can be determined at genomic level, by detecting the amount of mRNA that has been transcribed from said gene, or at proteomic level, by detecting the amount of protein encoded by said gene that has been translated in the cell.

Thus, in another embodiment of the first aspect of the invention, the determination of the level of expression of step (b) is carried out by determining the amount of the mRNA of said gene(s). Methods for determining the amount of mRNA of a particular gene in a sample are well known in the state of the art.

The polymerase chain reaction (PCR) is the most widely used method for the *in vitro* enzymatic amplification of nucleic acids, but it is not the only one. The ligase chain reaction (LCR), for example, can be used for the sensible detection of a DNA sequence with an increased specificity as compared to PCR (LCR can be used for the discrimination among alleles). During LCR, for each of the two DNA strands, two partial probes are ligated to form the actual one; thus, LCR uses two enzymes: a DNA polymerase and a DNA ligase. Each cycle results in a doubling of the target nucleic acid molecule.

A quantitative method for the determination of nucleic acids is real time PCR. Real time PCR, also called quantitative real time PCR (qPCR), is used to amplify and simultaneously quantify a targeted DNA molecule. The procedure follows the general principle of polymerase chain reaction; its key feature is that the amplified DNA is quantified as it accumulates in the reaction in real time after each amplification cycle. Two common methods of quantification are the use of fluorescent dyes that intercalate with double-stranded DNA, and modified DNA oligonucleotide probes that fluoresce when hybridized with a complementary DNA.

Further strategies have been developed based on the original PCR for the amplification of RNA, such as reverse transcription polymerase chain reaction (RT-PCR). In this technique, the RNA strand is first reverse transcribed into its DNA complement or complementary DNA, followed by amplification of the resulting DNA using polymerase chain reaction. Another method in molecular biology which is used to amplify RNA sequences is Nucleic Acid Sequence Based Amplification (NASBA). Explained briefly, NASBA works as follows: (a) RNA template is given to the reaction mixture and the first primer attaches to its complementary site at the 3' end of the template; (b) reverse transcriptase synthesizes the opposite, complementary DNA strand; (c) RNAse H destroys the RNA template (RNAse H only destroys RNA in RNA-DNA hybrids, but not single-stranded RNA); (d) the second primer attaches to the 5' end of the DNA strand and 5- T7 RNA polymerase produces a complementary RNA strand which can be used again in step (a). NASBA has been introduced into medical diagnostics, where it has been shown to give quicker results than PCR, and it can also be more sensitive. Both reverse transcription PCR and NASBA are both suitable techniques for the determination of gene expression.

Preferably, the level of expression of the genes is determined by RT-PCR. More preferably, the method for determining the level of expression of the genes is quantitative reverse transcription PCR.

Other suitable techniques for the determination of gene expression are macroarray screening, microarray screening and nanoarray screening.

In another embodiment of the first aspect of the invention the level of expression of the selected genes(s) is determined by the amount of the protein(s) encoded by said gene(s).

Several methods for determining the amount of a particular protein in a sample are well known in the state of the art. Generally, these methods will make use of binding moieties.

By "binding moiety" is meant a molecule or molecule segment capable of binding specifically to the target protein (in the present case the target protein is the protein encoded by the gene from which its expression is determined). Preferably the binding moiety can be a polypeptide molecule (such as an antibody or a fragment thereof) or a nucleic acid aptamer, among others.

Polypeptide binding moieties can be identified by means of a screening method. A suitable screening method for identifying peptides or other molecules which selectively bind a target protein may comprise contacting the target protein with a test peptide or other molecule under conditions where binding can occur, and then determining if the test molecule or peptide has bound the target protein or peptide. Methods of detecting binding between two moieties are well known in the art of biochemistry. The most frequently used polypeptide binding molecules are antibodies.

By "antibody" is meant a whole antibody, including without limitation a chimeric, recombinant, transgenic, humanised, grafted and single chain antibody, and the like, as well as any fusion protein, conjugates, fragments, or derivates thereof that contain one or more domains that selectively bind the target protein or peptide. An antibody fragment means an Fv, a disulfide linked Fv, scFv, Fab, Fab', or F(ab')2 fragment, which are well known in the art. There are various advantages for using antibody fragments, rather than whole antibodies. For example, the smaller size of the fragments may lead to an improved penetration to the target site, and effector functions of whole antibodies, such as complement binding, are removed. Fab, Fv, ScFv and dAb antibody fragments can be expressed in and secreted from E. coli, thus allowing the facile production of large amounts of said fragments. When whole antibodies are used for the present invention, monoclonal antibodies are preferred.

As mentioned above, aptamers are also preferred binding moieties. Aptamers are nucleic acid molecules that are designed in such a way that they can bind to desired target molecule (in the present case the target molecule is the protein encoded by the gene from which its expression is determined) and show nuclease resistance. The aptamers can be synthesized through repeated rounds of *in vitro* partition, selection and amplification, a methodology known in the state of the art as "SELEX". Alternatively, they can be synthesized, for example, by step-wise solid phase.

Binding moieties, such as antibodies, are frequently employed for the determination of the expression of a particular protein within a cell by immunohistochemical or immunofluorescent techniques. Preferably, the technique to be used in the present invention is inmunohistochemistry (IHC). IHC is a well known technique that is widely used to understand the distribution and localization of biomarkers and differentially expressed proteins in different parts of a biological tissue.

In a second aspect, the present invention provides a kit for carrying out the method according to the first aspect of the invention, which comprises adequate means for determining the level of expression of at least one gene selected from the group consisting of: RNGTT, HDGFRP3, FARP1, HMGB3, LGALS3BP, PON2, CDK2AP1, DBN1, CNR1, CNN3, SOX11, SEMAR, and CSNK1E. Preferably, the kit comprises adequate means for determining the level of expression of at least one gene selected from the group consisting of: SOX11, HDGFRP3, CNN3, FARP1, PON2, DBN1 and HMGB3. More preferably, the kit comprises adequate means for determining the level of expression of at least one gene selected from the group consisting of: SOX11, HDGFRP3 and CNN3. Still more preferably, the kit comprises adequate means for determining the level of expression of SOX11.

The kit can include specific means for the determination of the mRNA of the gene(s) by quantitative reverse transcription PCR. The kit can then contain all the required reagents and controls to perform quantitative reverse transcription PCR, including oligonucleotides suited as primers for the amplification of the selected genes.

In another embodiment of the second aspect of the invention, the kit contains adequate means for determining the amount of protein encoded by at least one of the genes mentioned above.

The kit of the invention can contain binding moieties that specifically bind to the proteins encoded by the disclosed genes and also additional means to perform the determination of said proteins. For instance, if the selected technique is western blot, the kit can also contain suitable buffers and reagents for electrophoresis and blotting, as well as developing reagents and control samples, such as protein ladders. If the technique to be used is IHC the kit can additionally contain reagents and controls to perform IHC.

The kit provided by the present invention can be used in a routine clinical practice to identify patients that suffer from a MCL with an indolent clinical course, thus differentiating said patients from other patients that suffer from a more conventional and aggressive form of the disease. With the kit of the invention the clinicians will be able to apply more individualized and risk-adapted treatment strategies to patients suffering from MCL.

In a third aspect, the present invention refers to the use of SOX11 as a marker for the prognosis of a mantle cell lymphoma with an indolent clinical course.

Throughout the description and claims the word "comprise" and variations of the word, such as "comprising", is not intended to exclude other technical features, additives, components, or steps. Additional objects, advantages and features of the invention will become apparent to those skilled in the art upon examination of the description or may be learned by practice of the invention. The following examples and drawings are provided by way of illustration, and are not intended to be limiting of the present invention. Furthermore, the present invention covers all possible combinations of particular and preferred embodiments described herein.

### BRIEF DESCRIPTION OF THE DRAWINGS

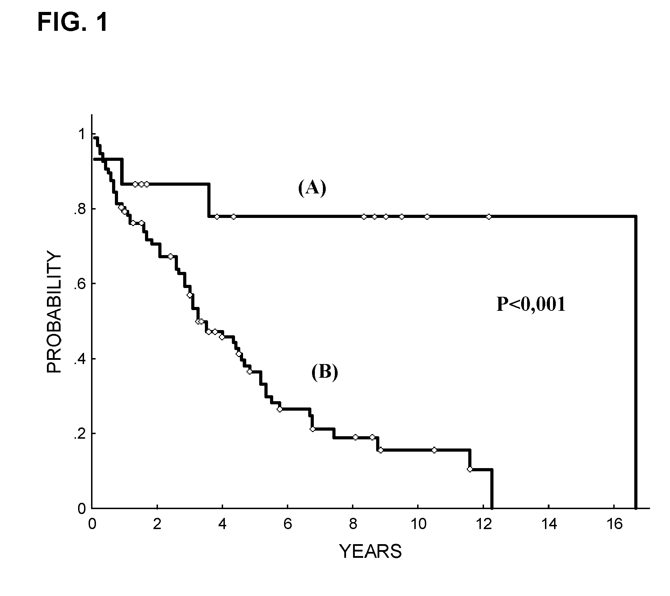

FIG. 1: Kaplan-Meier estimates of overall survival of 112 patients with MCL in the validation series according to SOX11 expression as determined by immunohisstochemistry on formalin-fixed, paraffin-embedded tumoral tissues. Each line represents a subgroup of MCL patients as defined by SOX11 expression: A) patients with SOX11 negative expression (n=15, dead: 4); and B) patients with SOX11 positive expression (n=97, dead: 68).

### EXAMPLES

### A) Study population

Twelve patients diagnosed with MCL (from May 1994 to August 2005) who showed an indolent clinical course for more than two years (median 6.4 years; range 2.5 to 10.4) without chemotherapy were selected for the study. These cases were called indolent MCL (iMCL) and were compared to 15 conventional MCL (cMCL) that required chemotherapy at diagnosis and had peripheral blood tumor cells available. All the cases had the t(11;14) and expressed cyclin D1. Ten of the 12 iMCL had been initially diagnosed as splenic marginal zone lymphoma (SMZL) (4 cases), chronic lymphocytic leukemia (CLL) (4 cases), or leukemic lymphoid neoplasm, not otherwise specified (2 cases) and reclassified as MCL during the evolution of the disease when a t(11;14) translocation and cyclin D1 overexpression were identified. The two additional patients were diagnosed with an incidental "in situ" MCL detected in a lymph node biopsy in which the cyclin D1 positive cells were restricted to the mantle zone of otherwise reactive follicles. In spite of the diagnosis of MCL, none of these 12 patients was considered for chemotherapy because of the evidence of an indolent stable disease from the moment of the initial diagnosis to the reclassification as MCL, or because the limited disease. The main characteristics of the 12 iMCL and 15 cMCL are summarized in Table 1.

| Table 1: Clinical and pathological characteristics of the 12 iMCL and 15 cMCL | | | | |
|---|---|---|---|---|
| | | **iMCL** **(n=12)** | **cMCL** **(n=15)** | **P value** |
| **Clinical and pathological data**† | | | | |
| Median age (range) | | 58(41-75) | 67(30-83) | NS |
| Sex (Male / Female) | | 9/3 | 11/4 | NS |
| B symptoms (%) | | 0 | 33 | 0.03 |
| ECOG≥2 (%) | | 0 | 70 | 0.01 |
| Nodal presentation (lymph nodes >1 cm) (%)* | | 17 | 93 | <0.001 |
| Palpable splenomegaly (%) | | 50 | 60 | NS |
| GI involvement (%)† | | 100 | 50 | NS |
| Bone marrow involvement (%)† | | 92 | 91 | NS |
| WBC count >10x10⁹/L (%)† | | 33 | 82 | NS |
| Lymphocyte count >5x10⁹/L (%)† | | 44 | 82 | NS |
| Atypical lymphocytes (%) | | 92 | 91 | NS |
| High serum LDH* (%)† | | 0 | 46 | 0.03 |
| High serum β₂microglobulin (%)† | | 20 | 80 | NS |
| Intermediate or high-risk MIPI (%)† | | 0 | 46 | 0.016 |
| Morphology | | | | |
| Small cell (%) | | 67 | 13 | 0.007 |
| Classical | | 33 | 74 | |
| Blastoid | | - | 13 | |
| CD5+ (%)† | | 64 | 93 | NS |
| *IGHV* gene hypermutations (>5%)† Genomic profile† | | 70 | 20 | < 0.04 |
| 0-1 imbalance | | 100 | 13 | <0.001 |
| ≥ 2 imbalances | | 0 | 87 | |

| **Evolutive data** | | | | |
|---|---|---|---|---|
| Splenectomy (%) | | 42 | 20 | |
| Chemotherapy at any time (%) | | 17 | 100 | |
| Median Follow-up in years (range)‡ | | 6.4 (2.5-10.4) | 3.3 (1.5-5.1) | NS |
| Dead patients (%) | | 0 | 47 | <0.001 |
| 5-year overall survival (%) | | 100 | 49 | 0.03 |
| iMCL: Indolent mantle cell lymphoma; cMCL: Conventional mantle cell lymphoma; NS: not significant; MIPI: Mantle cell lymphoma internationa prognostic index | | | | |
| * The two iMCL patients with nodal involvement had an isolated lymph node in the cervical region. | | | | |
| † Gastrointestinal (GI) involvement was assessed in 8 cMCL and 4 iMCL patients; Mantle cell lymphoma international prognostic index (MIPI) (cf. Hoster E., et al, "A new prognostic index (MIPI) for patients with advanced-stage mantle cell lymphoma", Blood 2008, vol. 111, p. 558-65) was determined in 13 cMCL and 8 iMCL; IGVH mutations and SNP-array were studied in 10 and 7 iMCL, respectively and all cMCL; Peripheral blood counts, LDH, β₂microglobulin, bone marrow involvement, and CD5 expression could be assessed in 23, 21, 18, 20 and 25 cases, respectively. | | | | |
| ‡ Follow-up of surviving patients. | | | | |

Patients with iMCL had leukemic disease (11 patients) and no evidence (10 patients) or only one isolated lymphadenopathy (>1 cm) (2 patients). The main characteristics of the patients are listed in Table 1 above. Splenectomy was performed in 5 patients and none received chemotherapy at diagnosis. After a median follow-up of 6.4 years, two patients showed disease progression at 5 and 7 years after diagnosis. One patient received chlorambucil and eventually was splenectomized. The other developed peripheral lymphadenopaty and gastrointestinal involvement and was treated with rituximab-chlorambucil, reaching a complete response.

### B) Gene expression analysis

Mononuclear cells were isolated from peripheral blood of 7 iMCL and the 15 cMCL by gradient centrifugation, frozen in dimethyl sulfoxide (DMSO) and stored in liquid nitrogen until analysis. Tumor cells were purified (>98% as determined by flow cytometry) using anti-CD19 magnetic microbeads (Miltenyi Biotech^{®}, Bergisch Gladbach, Germany). Peripheral blood tumor cells were also purified from 17 CLL, 7 follicular lymphoma (FL), 4 SMZL, 3 hairy cell leukemia (HCL), and 2 HCL-variant (HCLv) diagnosed according to the WHO classification (cf. Swerdlow S.H. et al., (Eds) "WHO Classification of Tumours of Haematopoietic and Lymphoid Tissues", 2008, Fourth Edition. Lyon: IARC press).

Total RNA was extracted from the purified tumor cells with the TRlzol^{®} reagent following the recommendations of the manufacturer (Invitrogen Life Technologies^{®}, Carlsbad, CA, USA). RNA integrity was examined with the Agilent 2100 Bioanalyser (Agilent Technologies^{®}, Palo Alto, CA, USA) and only high quality RNA samples were hybridized to Affymetrix GeneChip^{®} Human Genome U133 plus 2.0 (Affymetrix^{®}) arrays, according to Affymetrix standard protocols. The analysis of the scanned images and the determination of the signal value for each probe set of the array were obtained with the GeneChip^{®} Operating Software (GCOS, Affymetrix^{®}). Data normalization was performed by the global scaling method with a target intensity set at 150. The data analysis was performed with the DNA-Chip analyzer software 2006 (dChip, Boston, MA, USA) and BRB-Array Tools, v.3.6.0 software^{®} (cf. Simon R.M. et al., "Design and Analysis of DNA Microarray Investigations", 2004 New York: Springer-Verlag). In order to perform an unsupervised analysis of all B-NHL cases, genes were filtered according to the standard deviation (SD) across samples (1 <SD<1000) and the expression level (log2 signal ≥ 7 in 1 10% samples). In total 3644 probe-sets were retained after applying the filtering criteria described above, and were used for the clustering analysis using centered correlation as distance metric and average linkage. The robustness of the clusters was evaluated using the R (reproducibility) measure described by McShane et al. (cf. "Methods for assessing reproducibility of clustering patterns observed in analysis of microarray data", Bioinformatics 2002, vol. 18, p. 1462-9) and implemented in the BRB-Array Tools. A R of 1 means perfect reproducibility of that cluster, and a R of 0 means no reproducibility of the cluster. For the supervised analysis of MCL samples, gene detection calls were determined with the dChip software and only the probe sets that were present in at least 75% of either indolent and conventional MCL cases were considered for further analysis. In addition, an arbitrary value of 10 before log2 transformation was assigned to the probe-sets with an expression below 10 before log2 transformation. In total, 22732 probe-sets were used for differential gene expression analysis using the Significance Analysis of Microarray Data (SAM) method implemented in BRB-Array Tools (cf. Simon R.M. *et al., supra*). Two levels of significance were used for detecting differentially expressed genes, considering a median False Discovery Rate (FDR) < 0.1 and a go^{th} percentile FDR < 0.1.

The molecular proliferation signature value of the tumors was calculated as described by Rosenwald et al. (cf. "The proliferation gene expression signature is a quantitative integrator of oncogenic events that predicts survival in mantle cell lymphoma", Cancer Cell 2003, vol. 3, p. 185-97) using the 18 out of the 20 genes of the proliferation signature that were present in the Affymetrix GeneChip^{®} Human Genome U133 plus 2.0. When more than one probe set was present for a specific gene the inventors used the mean as the gene expression value for that gene. Gene expression values for each gene were standardized across samples and the mean of all eighteen genes was reported as a proliferation signature value for each sample.

To determine the molecular relationship of iMCL with cMCL and other leukemic lymphoid neoplasms the inventors compared the gene expression profile of 7 iMCL, 15 cMCL, 17 CLL, 7 FL, 4 SMZL, 3 HCL and 2 HCLv. The unsupervised hierarchical clustering analysis revealed that all samples were grouped according to their diagnosis in four main clusters corresponding to SMZL/HCL/HCLv, CLL, MCL, and FL (data not shown). iMCL and cMCL were grouped together in a robust cluster (R-index=0.997). This result supports the idea that iMCL and cMCL share a common gene expression profile that is different from other leukemic lymphoid neoplasms.

The MCL cluster showed an asymmetric distribution of the cMCL and iMCL in two sub-clusters. The inventors performed a supervised analysis to identify differentially expressed genes between the two subtypes using the significance analysis of microarray data (SAM) algorithm. When the inventors considered a median False Discovery Rate (FDR) < 0.1, 569 probe-sets were selected as differentially expressed (data not shown). A more stringent analysis (90^{th} percentile FDR < 0.1) identified 23 probe-sets corresponding to thirteen annotated genes: RNGTT, HDGFRP3, FARP1, CSNK1E, SETMAR, HMGB3, LGALS3BP, PON2, CDK2AP1, DBN1, CNR1, CNN3, and SOX11 (Table 2). These genes were strongly expressed in 13 of the 15 cMCL and underexpressed in all iMCL.

| Table 2: List of differentially expressed genes between iMCL and cMCL cases | | | | | |
|---|---|---|---|---|---|
| **Gene** | **Probe set** | **GMI*** **iMCL** | **GMI*** **cMCL** | **GMI*** **Ratio** | **P-value** |
| *RNGTT* | 211849_s_at | 138.15 | 814.45 | 0.17 | 6.20 x10⁻⁶ |
| *HDGFRP3* | 209524_at | 10.66 | 265.27 | 0.04 | 1.23 x10⁻⁵ |
| *FARP1* | 239246 at | 16.53 | 160.82 | 0.10 | 1.51 x10⁻⁵ |
| *HMGB3* | 225601_at | 20.86 | 130.16 | 0.16 | 1.57 x10⁻⁵ |
| *LGALS3BP* | 200923_at | 58.71 | 385.44 | 0.15 | 4.69 x10⁻⁵ |
| *PON2* | 210830_s_at | 16.13 | 183.59 | 0.09 | 4.93 x10⁻⁵ |
| *CDK2AP1* | 201938_at | 239.26 | 1008.01 | 0.24 | 5.16 x10⁻⁵ |
| *DBN1* | 202806_at | 13.78 | 110.52 | 0.12 | 8.40 x10⁻⁵ |
| *CNR1* | 1560225_at | 88.01 | 911.88 | 0.10 | 0.00014 |
| *CNN3* | 201445_at | 15.98 | 139.98 | 0.11 | 0.00026 |
| *SETMAR* | 206554_x_at | 52.50 | 150.67 | 0.35 | 0.00027 |
| *SOX11* | 204913_s_at | 33.52 | 691.44 | 0.05 | 0.00027 |
| *CSNK1E* | 202332_at | 158.63 | 342.79 | 0.46 | 0.00030 |
| * GMI: Geometric Mean of Intensities | | | | | |

Three of these genes, SOX11, HDGFRP3, and CNN3, were highly expressed in 13 of the 15 cMCL and virtually negative in all iMCL (P<0.001). No significant differences in the cyclin D1 levels or in the microarray based proliferation signature14 between iMCL and cMCL were detected (data not shown). The differences of expression between iMCL and cMCL were analysed with a T-test and by using the SPSS software package version 14.0 (SPSS^{®} Inc).

To validate the results of the microarrays expression data the inventors re-examined the mRNA levels of SOX11, HDGFRP3, and CNN3 by quantitative reverse transcription PCR. One µg of total RNA from the 7 iMCL and 15 cMCL cases was treated with Turbo DNAse (Ambion^{®} Inc, Austin, TX, USA). Complementary DNA (cDNA) was then synthesized using the Superscript III™ system (Invitrogen^{®}) and following manufacturer instructions. The gene expression levels were determined with the following pre-developed assays (Applied Biosystems^{®}): SOX11 Hs00846583_s1; HDGFRP3 Hs01016437_m1; and CNN3 Hs00156565_m1. Expression levels were calculated with the 2-^{ΔΔCt} method using human β-glucoronidase (GUS) as endogenous control and Jurkat cell line as mathematical calibrator (cf. Livak K.J. et al., "Analysis of relative gene expression data using real-time quantitative PCR and the 2(-Delta Delta C(T)) Method", Methods 2001, vol. 25, p. 402-8). The correlation between microarray and quantitative reverse transcription-PCR measures were analysed with the Pearson correlation test. Microarray versus qRT-PCR levels was found to be R²=0.89, 0.93 and 0.97, for SOX11, HDGFRP3, and CNN3, respectively. This data confirm the correlation between results obtained for mRNA expression levels by microarrays and quantitative reverse transcription PCR.

### C) SOX11 protein expression by immunohistochemistry

The inventors searched for reliable antibodies to assess the protein expression of the differentially expressed genes between iMCL and cMCL on routinely processed biopsies and found only one antibody against SOX11. Therefore, tissue sections which had been used for the diagnosis of MCL in 12 cMCL and 11 iMCL could be investigated by immunohistochemistry.

Immunohistochemistry was performed on formalin-fixed, paraffin-embedded tissues with the EnVision+System Peroxidase (DAB) method (DAKO^{®}, Carpinteria, CA). The antigen retrieval was done by heating samples on ethylenediaminetetraacetic acid (EDTA) buffer in a microwave oven. Primary antibody against SOX11 (HPA000536, Atlas Antibodies^{®}, Stockholm, Sweden) was used at a 1/100 dilution. The immunohistochemical expression of SOX11 was only detected in formalin fixed tissue sections. Bone marrow biopsies and B-5 fixed tissues were not assessable. According to the original description, SOX11 protein expression was identified as a strong nuclear staining of the lymphoid cells.

Concordantly with the array data, the strong nuclear expression of SOX11 was detected in 11 of the 12 cMCL, whereas it was negative in the 6 iMCL examined previously by microarrays. The negative cMCL was also negative by mRNA (data not shown). The 5 additional iMCL that could not be studied by microarrays were also negative for the SOX11 protein. Thus, SOX11 mRNA and/or protein expression were negative or significantly underexpressed in all iMCL whereas it was highly expressed in 13 of the 15 cMCL.

### D) SOX11 protein expression in an independent MCL series

To confirm whether SOX11 could be a biomarker to recognize two subtypes of MCL with different clinicopathological features and outcome, the inventors investigated by immunohistochemistry the protein expression in an independent series of 112 patients diagnosed and managed as standard MCL regardless of the SOX11 status. The 112 patients had been diagnosed with MCL from 1986 to 2007 at the Hospital Clinic, Barcelona, Centro Nacional de lnvestigaci6n Oncol6gica, Madrid, Spain, and Institute of Pathology, Würzburg, Germany. These cases had available clinical information and formalin-fixed, paraffin-embedded tissue sections, which had been used for the diagnosis of MCL. Immunohistochemistry was performed in these tissues. The study was approved by the Institutional Review Board of the respective institutions.

SOX11 nuclear expression was detected in 97 (87%) tumoral tissues and was absent in 15 (13%). The main clinical and biological features of the patients according to SOX11 expression are summarized in Table 3. SOX11-negative patients had more frequently splenomegaly, higher white blood cell and lymphocyte counts than in SOX11-positive. Regarding the clinical presentation, eight patients showed a non-nodal presentation, with 7 of them (87%) being SOX11-negative and only 1 SOX11-positive (P<0.001).

| Table 3. Main clinical and pathological features of 112 patients with MCL according to SOX11 expression in the validation set | | | |
|---|---|---|---|
| | **SOX11** **negative** **(n=15)** | **SOX11** **positive** **(n=97)** | **P value** |
| **Clinical and pathological data** | | | |
| Median age (range) | 57(42-77) | 63(31-83) | NS |
| Sex (M/F) | 11/4 | 71/26 | NS |
| B symptoms (%) | 22 | 48 | NS |
| ECOG≥ 2 (%) | 20 | 28 | NS |
| Nodal presentation (lymph nodes >1cm) (%) | 53 | 99 | <0.001 |
| Palpable splenomegaly (%) | 92 | 48 | 0.005 |
| Bone marrow involvement* (%) | 83 | 87 | NS |
| Ann Arbor stage IV (%) | 92 | 74 | NS |
| WBC count >10x10⁹/L* (%) | 57 | 18 | 0.04 |
| Lymphocyte count >5x10⁹/L* (%) | 83 | 24 | <0.001 |
| High serum LDH* (%) | 22 | 29 | NS |
| High serum β₂microglobulin* (%) | 75 | 55 | NS |
| \|P\| intermediate/high or high-risk* (%) | 37 | 48 | NS |
| MIPI high-risk* (%) | 33 | 46 | NS |
| Ki67 high (>50%) (%) | 20 | 28 | NS |

| **Evolutive data** | | | |
|---|---|---|---|
| Median follow-up in years (range) † | 8.3 (1.3-12.2) | 3.8 (1-11.6) | NS |
| Front-line adriamycin-containing | 67 | 72 | NS |
| polychemotherapy (%) | | | |
| Complete response (%) | 40 | 54 | NS |
| 5-year time to treatment failure (%)‡ | 75 | 23 | 0.04 |
| 5-year overall survival (%) | 78 | 36 | 0.001 |
| NS: not significant; \|P\|- International prognostic index for aggressive lymphomas; MIPI: Mantle-cell lymphoma international prognostic index; | | | |
| * The number of assessable cases for bone marrow involvement, \|P\|, MIPI, peripheral blood counts, LDH, and β₂microglobulin were 82, 62, 71, 104, 61, and 50, respectively. | | | |
| † Surviving patients. | | | |
| ‡ In 78 patients with this information available | | | |

The median overall survival (OS) of the series was 4 years. SOX11-negative patients had a longer OS than those SOX11-positive (5-year OS: 78% (95% confidence interval [CI]: 56-100) vs. 36% (95% CI: 25-47), respectively; P=0.001) (FIG. 1). Since MCL with non-nodal presentation has been described as a possible indolent variant of this tumor (cf. Orchard J. *et al*., *supra*), the inventors examined whether the most important variable for OS was nodal/non-nodal presentation or SOX11 expression by means of a Cox analysis. Only SOX11 retained predictive value in this model (p<0.001; relative risk [RR]: 5.8). Finally, when the Ki67 proliferation index was incorporated to the Cox model, SOX11 (P=0.006; RR: 3.8) and Ki67 >50% (P=0.02; RR: 1.9) resulted the most important variables for OS.

The inventors found that a supervised comparison of the iMCL and cMCL gene expression profiles revealed 13 genes with highly significant differences, all of them overexpressed in cMCL and underexpressed in iMCL. These findings indicate that the expression of this signature may be very specific of cMCL since these genes were not expressed in iMCL or other leukemic lymphoid neoplasms. Moreover, lack of SOX11 expression identified a subset of patients with non-nodal presentation, higher lymphocyte counts, and longer survival similar to that of our initial iMCL. On the contrary, SOX11-positive MCL had a conventional nodal presentation and worse outcome. Moreover, the inventors also found that SOX11 expression was able to distinguish favorable MCL cases more consistently than the nodal presentation or the Ki67 index.

### E) Prognostic tool based on expression of SOX11, HDGFRP3, DBN1

The purpose of this study was designing a practical tool based on quantitative expression of a few number of genes by PCR in leukemic mantle cell lymphoma (MCL) samples, to facilitate the recognition of indolent MCL patients.

The method chosen for determining the level of expression of the mRNA of the selected genes was the quantitative reverse transcription PCR (qRT-PCR). Peripheral blood specimens of 43 MCL were analyzed, 17 of these samples chosen from the previous study. Furthermore, 37 leukemic B-cell neoplasms were analyzed, including 19 chronic lymphocytic leukemia (CLL), one of them from the previous study; 8 splenic marginal zone lymphoma (SMZL), 3 of them from the previous study; 5 hairy cell leukemia (HCL), 3 of them from the previous study; and 5 follicular lymphoma (FL), 2 of them from the previous study. The lymphoid B-cell population was at least 60% in 50 cases and in 30 cases the tumor B-cells were purified (>98% as determined by flow cytometry) using anti-CD19 magnetic microbeads (Miltenyi Biotech).

Total RNA was extracted from frozen peripheral blood using AllPrep DNA/RNA Mini Kit (Qiagen, Germantown, MA, USA). The potential residual DNA was removed using the TURBO DNA-free^{™} kit from Ambion (Applied Biosystems) according to the manufacturer's protocol. Complementary DNA synthesis was carried out from 500 ng of total RNA. It was denatured for 5 minutes at 65°C, and then reverse transcription was performed with 0.75 U/µl Moloney-murine leukemia virus reverse transcriptase (Invitrogen, Gaithersburg, MD) in the manufacturer's buffer with 0.75 U/µl of RNase inhibitor (Promega , Madison, WI) and 2.5 mM random hexamer primers at 37 °C for 1 hour in a final volume of 40 µll. Then, the product was amplified and quantified using TaqMan Universal Master Mix (Applied Biosystems, Foster City, CA, USA). TaqMan Gene Expression Assays were used for evaluating the expression of CCND1 (Hs00765553_m1) and DBN1 (Hs00365623 m1). Primer sets and fluorescent TaqMan^{®} MGB probes were designed in order to create a shorter amplicon than the one commercialized for SOX11 and HDGFRP3 with Primer Express^{®} Software Version 2.0 (Applied Biosystems). An 87 bp amplicon length for SOX11 was obtained (probe: 5'-TTTTAACCACGGATAATTG-3' (SEQ ID NO: 1), primer forward: 5'-CATGTAGACTAATGCAGCCATTGG-3' (SEQ ID NO: 2), primer reverse: 5'-CACGGAGCACGTGTCAATTG-3' (SEQ ID NO: 3)) and a 62 bp amplicon for HDGFRP3 (probe: 5'-CGGGCAACGACACAA-3' (SEQ ID NO: 4), primer forward: 5'-GCAGCTCTGAGGGTGGAGAT-3' (SEQ ID NO: 5), primer reverse: 5'-TTTCTGCAAGTCTGAAGTTGTGTTT-3' (SEQ ID NO: 6)).

Relative quantification of gene expression levels was analysed with the 2-^{ΔΔCt} method using human β-glucoronidase (GUS) as an endogenous control and universal human reference RNA (Stratagene, Agilent Technologies, Santa Clara, CA, USA) as the mathematical calibrator. Moreover, the mutational status of the immunoglobulin heavy-chain variable-region (IGVH) was examined in 32 of the 43 (74%) MCL.

The relationship between the mRNA expression of 7 (SOX11, HDGFRP3, HMGB3, DBN1, PON2, CDK2AP1 and RNGTT) of the 13 genes forming the reported signature detected by microarrays and qRT-PCR was initially examined. The 3 genes with the highest correlation coefficient (SOX11, HDGFRP3, DBN1) were selected for the clinical study. Two subgroups of MCL were segregated based on the expression levels of these genes using Ward's unsupervised hierarchical clustering method. One group (25 cases) had high expression of SOX11, HDGFRP3 and DBN1 whereas these genes were absent or negligible in the other subgroup (18 cases). MCL patients with high expression of these genes had a significant shorter survival than patients with lower expression (4-year OS: 58% vs 90%; P=0.008). These two groups of MCL also differed in their *IGVH* mutational status. The subgroup of MCL with better survival and low expression of these genes had more frequent hypermutated *IGVH* genes (≥5% mutations) (12 of 15 (80%)) whereas tumors with high expression had unmutated *IGVH* (≤2% mutations) (14 of 17, 82%) (P<0.01). Cyclin D1 expression levels were similar in both subgroups of MCL. Cyclin D1, SOX11, HDGFRP3, and DBN1 mRNA expression were negative in the 37 non MCL lymphoid neoplasms.

It is concluded that the low expression of SOX11, HDGFRP3, and DBN1 combined with the expression of Cyclin D1 determined by qRT-PCR identifies a subgroup of leukemic MCL with predominantly hypermutated *IGVH* genes and a very indolent clinical outcome. This assay may be useful to guide the selection of therapy in leukemic MCL.

## Claims

1. A method for the prognosis of a mantle cell lymphoma with an indolent clinical course, comprising the steps of:
a) providing a sample from a patient suffering from mantle cell lymphoma;
b) determining the level of expression of at least one gene selected from the group consisting of: RNGTT, HDGFRP3, FARP1, HMGB3, LGALS3BP, PON2, CDK2AP1, DBN1, CNR1, CNN3, SOX11, SEMAR and CSNK1E in said sample; and
c) comparing the level of expression of each of the measured genes with respect to the level of expression of the same genes in a control sample;
wherein the absence of expression or the underexpression of said genes with respect to the same genes in said control sample is indicative of the indolent clinical course of the mantle cell lymphoma.

2. The method according to claim 1, wherein the expression level is determined for at least one gene selected from the group consisting of: SOX11, HDGFRP3, CNN3, FARP1, PON2, DBN1, and HMGB3.

3. The method according to claim 2, wherein the expression level is determined for at least one gene selected from the group consisting: of SOX11, HDGFRP3, and CNN3.

4. The method according to claim 3, wherein the expression level of SOX11 is determined.

5. The method according to any of the preceding claims, wherein the determination of the level of expression of step (b) is carried out by determining the amount of the mRNA of said gene(s).

6. The method according to claim 5, wherein the determination is carried out by quantitative reverse transcription PCR.

7. The method according to any of the claims 1-4, wherein the determination of the level of expression of step (b) is carried out by determining the amount of the protein encoded by said gene(s).

8. The method according to claim 7, wherein the determination is carried out by inmunohistochemistry.

9. A kit for carrying the method as defined in any of the claims 1-8, which comprises adequate means for determining the level of expression of at least one gene selected from the group consisting of: RNGTT, HDGFRP3, FARP1, HMGB3, LGALS3BP, PON2, CDK2AP1, DBN1, CNR1, CNN3, SOX11, SEMAR and CSNK1E.

10. The kit according to claim 9, which comprises adequate means for determining the level of expression of at least one gene selected from the group consisting of: SOX11, HDGFRP3, CNN3, FARP1, PON2, DBN1, and HMGB3.

11. The kit according to claims 10, which comprises adequate means for determining the level of expression of at least one gene selected from the group consisting: of SOX11, HDGFRP3, and CNN3.

12. The kit according to claim 11, which comprises adequate means for determining the level of expression of SOX11.

13. The kit according to any of the claims 9-12, which comprises adequate means for determining the amount of protein encoded by said gene(s).

14. The kit according to any of the claims 9-12, which comprises adequate means for determining the amount of mRNA of said gene(s).

15. The kit according to claim 14, which comprises substances required for carrying out quantitative reverse transcription PCR.

16. Use of SOX11 as a marker for the prognosis of a mantle cell lymphoma with an indolent clinical course.
